# EUROPEAN PATENT SPECIFICATION

(11) **EP 0 746 327 B1**
(45) Date of publication and mention of the grant of the patent: **21.04.2004**
(21) Application number: 95908347.8
(22) Date of filing: 17.02.1995
(51) Int. Cl.: A61K 33/00, A61K 47/12, A61P 31/04

(54) **ACIDIFIED NITRITE AS AN ANTIMICROBIAL AGENT**
ANGESÄUERTES NITRIT ZUR VERWENDUNG ALS ANTIMIKROBIELLES MITTEL
NITRITE ACIDIFIE EN TANT QU'AGENT ANTIMICROBIEN

(30) Priority: 21.02.1994 GB 9403284; 07.03.1994 GB 9404365
(43) Date of publication of application: 11.12.1996
(73) Proprietor: ABERDEEN UNIVERSITY, Aberdeen AB2 1RY (GB)
(72) Inventor: BENJAMIN, Nigel, Aberdeen AB2 1RQ (GB); DOUGALL, Hamish, Aberdeen AB1 7YB (GB)
(74) Representative: Lord, Hilton David
(86) International application number: PCT/GB1995/000338
(87) International publication number: WO 1995/022335

(56) References cited:
- US-A- 4 191 750
- DATABASE WPI Week 8426 Derwent Publications Ltd., London, GB; AN 84-161228 & JP,A,59 085 278 (AGENCY OF IND. SCI. TECH.) , 17 May 1984
- DATABASE WPI Week 8731 Derwent Publications Ltd., London, GB; AN 87-217091 & JP,A,62 142 559 (SHOKO KK) , 25 June 1987

## Description

The present invention relates to acidified nitrite as an antimicrobial agent.

Although nitrite has been used as a preservative for food for many years the mechanisms by which it kills microorganisms has not been elucidated. We have now found that nitrite in low concentration is effective in reducing the populations of bacteria, fungi and viruses on the animal body when pH is below 4. We believe that this mechanism is used by mammals to destroy swallowed microorganisms.

An active entero-salivary circulation in man provides a continuous flow of nitrate into the mouth where it is rapidly reduced to nitrite by bacteria on the tongue. The effect of salivary nitrate excretion is to provide a precursor for the generation of nitrogen oxides by the break down of the nitrite.

In brief we have found that exposure of a yeast, *Candida albicans* and the bacterium *E coli* to concentrations of nitrite in saliva together with acid condition similar to those found in the stomach for one hour caused a dose-dependent reduction in their survival. It is apparent therefore that the generation of nitrogen oxides and/or nitrous acid in the mouth and in the gastrointestinal tract, particularly the upper gastrointestinal tract, from acidified nitrite is preventative of microbial infection.

In the mouth bacteria rapidly reduce nitrates to nitrites. Once swallowed the acid conditions of the stomach protonate the nitrite to form nitrous acid (pKₐ approx 3.5). The nitrous acid in turn dissociates to form oxides of nitrogen as shown below.

NO₂ - + H+ = HNO₂ (1)

2HNO2 = H₂O + N₂O₃ (2)

N₂O₃ = NO + NO₂ . (3)

Endogenous and dietary nitrate is actively concentrated by salivary glands to more than 10 times the concentration in plasma and secreted in saliva. Thus the saliva provides a continuous source of nitrate to the upper gastrointestinal tract. Oral conversion of nitrate to nitrite is rapid and is restricted to the surface of the tongue in man and to the posterior third of the tongue in the rat.

The function of the entero-salivary circulation of nitrate is not known but it may well be that gastric acid by itself is not always sufficient to destroy many ingested micro-organisms and that the primary role of salivary nitrate secretion and conversion to nitrite is as a precursor for nitrogen oxides in the lumen of the stomach which will kill swallowed microorganisms.

The above identified mechanism is also applicable to the destruction of micro-organisms on the skin. For example athlete's foot or tinea pedis.

We have found that nitrite at concentrations of up to 4% in an inert carrier cream or ointment when mixed with an organic acid such as salicylic acid reacts to produce oxides of nitrogen which are effective in killing infectious organisms on the skin including fungi, yeast, bacteria and viruses. The combination of nitrite and acid causes mild erythema (redness) of the skin due to release of nitric oxides but this causes no significant inflammation.

The above identified mechanism is also useful in the sterilisation of objects such as dentures by utilising a sterilizing nitrite solution. Conventional solutions which are effective in sterilising dentures often taste unpleasant due to chlorine-based disinfectants. A combination of nitrite and acid results in a antimicrobial solution which has little or no taste. Other objects such as contact lenses may be sterilised in the same way.

Gastroenteritis continues to be a major problem in rearing pigs and other farm animals. Enteropathogenic *Escherichia coli* (especially those bearing the KBB antigen) are particularly implicated. Although gastric acidity is thought to be one of the main host defence systems which provides a barrier to orally-acquired infection, this is clearly ineffective in preventing organisms from reaching the more distal intestine in these animals.

Accordingly, in a first aspect of the present invention, there is provided an antimicrobial dosage form which comprises;
a pharmaceutically acceptable acidifying agent and a pharmaceutically acceptable source of nitrite or a nitrate precursor for said source of nitrite,
both said acidifying agent and said source of nitrite or nitrate precursor therefor being separately disposed in a respective pharmaceutically acceptable carrier or diluent for admixture to release nitrite at the intended environment of use,
said carrier or diluent being a cream or ointment, and
wherein the acidifying agent is present in an amount sufficient to reduce the pH at the environment of use to below pH4.

In an alternative aspect, there is provided an antimicrobial dosage form which comprises;
a pharmaceutically acceptable source of nitrite or a nitrate precursor for said source of nitrite,
and a pharmaceutically acceptable carrier or diluent therefor,
the dosage form being acidified by means of an acidifying agent which is a pharmaceutically acceptable organic acid,
and wherein the nitrite, when mixed with the acid, produces antimicrobially effective oxides of nitrogen, the acid being present in an amount sufficient to reduce the pH at the environment of use to below pH4, and the nitrite is present in concentrations of up to 4%.

The dosage forms of the present invention are preferably for use in the treatment of a viral condition, a fungal condition, or a bacterial condition.

The invention is characterised in that said acidifying agent and said source of nitrite ions or precursor therefor are respectively disposed in a respective cream, ointment, tablet or liquid for administration to release nitrite at the intended environment of use, and in that the acidifying agent is present in an amount sufficient to reduce the pH on administration of the dosage form to the environment of use to below pH4. Preferably the acidifying agent is an organic acid, for example salicylic acid or ascorbic acid. The precursor for the nitrite ion may be an alkali metal or alkaline earth metal nitrate capable of conversion to a nitrite by enzymic action.

In a further aspect of the invention there is provided a method of sterilising an object, which method comprises the steps of:-
1) preparing a pharmaceutically acceptable acidifying agent and a pharmaceutically acceptable source of nitrite or a nitrate precursor therefor,
2) admixing said acidifying agent with said source of nitrite in a liquid carrier or diluent in contact with said object, thereby to reduce the pH to below 4 to release sterilant nitrite to sterilise said object.

In a further form of the invention there is provided a sterilant composition comprising a pharmaceutically acceptable acidifying agent,
a pharmaceutically acceptable form of nitrite or a precursor therefor, and
a pharmaceutically acceptable carrier or diluent therefor, wherein the acidifying agent is adapted to reduce the pH at the environment of use to below 4.

In a still further form of the invention there is provided an animal feed supplement comprising separately a pharmaceutically acceptable acidifying agent to ensure that the pH at the environment of use is below pH 4, and a pharmaceutically acceptable source of nitrite ions or a nitrate precursor therefor, both in an amount sufficient to together produce a beneficial anti-bacterial effect at the environment of use, but insufficient to produce an adverse reaction in a target animal.

The present invention further provides use of a pharmaceutically acceptable organic acid, as defined herein, and a pharmaceutically acceptable source of nitrite or a nitrate precursor for said source of nitrite, as defined herein, in the manufacture of an antimicrobial medicament, as defined herein, wherein the nitrite, when mixed with the acid, produces antimicrobially effective oxides of nitrogen, the acid being present in an amount sufficient to reduce the pH at the environment of use to below pH4, the nitrite being present in concentrations of up to 4%.

The present invention further provides use of a pharmaceutically acceptable acidifying agent, as defined herein, and a pharmaceutically acceptable source of nitrite or a nitrate precursor for said source of nitrite, as defined herein, in the manufacture of an antimicrobial medicament, as defined herein,
both said acidifying agent and said source of nitrite or nitrate precursor therefor being separately disposed in a respective pharmaceutically acceptable carrier or diluent for admixture to release nitrite at the intended environment of use,
said carrier or diluent being a cream or ointment, and
wherein the acidifying agent is present in an amount sufficient to reduce the pH at the environment of use to below pH4.

The acidifying agent may be salicylic or ascorbic acid as above, and the source of nitrite ions or nitrate precursor therefor may be in an inorganic nitrate as set forth above. Where the animal is the pig, the supplement should be included in an amount sufficient to ensure that each adult animal will receive a balanced dose of inorganic nitrate of between 0.3 to 5.0 g/day and preferably about 1 g/day.

The invention will now be described, by way of illustration only, with reference to the following examples and figures accompanying the specification.

Figure 1 shows a diagram indicative of the effect of exposure to nitrate and differing hydrogen ion concentrations on the survival of *C albicans* where the vertical axis is the optical density in absorbance units and the horizontal axis is the pH.

Figure 2 shows growth curves of *E coli* following exposure to acid alone or acid with a nitrite where the vertical axes are optical density in absorbance units and the horizontal axes are time in hours.

Figure 3 shows growth curves of *E coli* following exposure to pH3 in various nitrite concentrations where the vertical axis shows the optical density in absorbance units and the horizontal axis is time in hours.

Figure 4 shows the generation of nitric oxide from sodium nitrite at different levels of acidity where the vertical axis is the nitric oxide concentration (nM) and the horizontal axis is pH.

### EXAMPLE 1

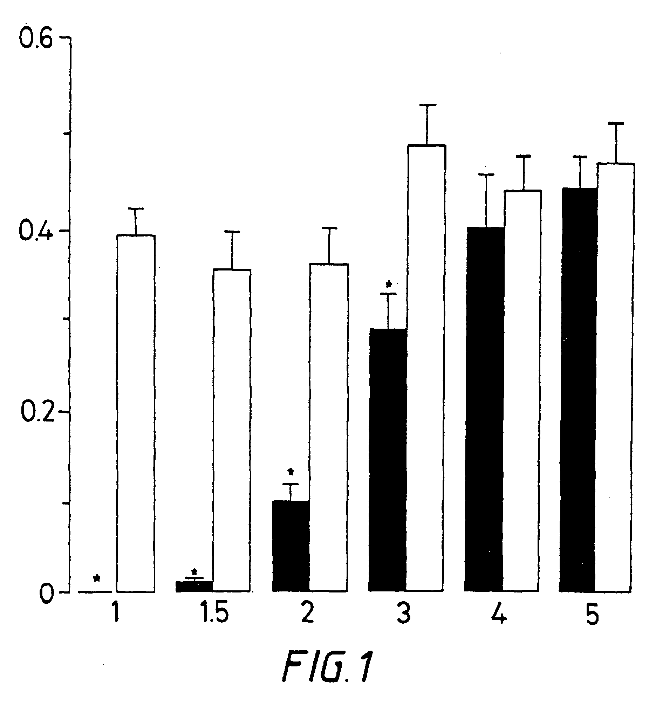

with reference to Figure 1 a single colony of C albicans was used to inoculate an overnight culture in Sabouraud's broth. 10µl of this broth was added to 940µl of a citrate/phosphate buffered Sabouraud's broth to which was added sodium nitrite (50µl; final concentration 250µM) or distilled water as a control. After one hour incubation at 37°C, 10µl was removed and cultured in 190µl standard Sabouraud's broth with continual agitation (Gallenkamp orbital incubator) in a 96-well microtitre plate at 37°C. Growth was monitored by measurement of optical density at 570nm at regular time intervals. The results are a mean of 16 separate experiments.

The effect of exposure to nitrite and differing hydrogen ion concentrations on the survival of C albicans is shown in Figure 1. The open bars show the growth of C albicans measured by the optical density method following exposure to acid alone for 1 hour, while the closed bars show growth following exposure to acid and 250µM sodium nitrite. There is a significant difference from the control at p>0.05 (Mann-Whitney U test). It is apparent therefore that the incubation of C albicans in acid alone for one hour had little effect on the number of viable organisms subsequently grown, whereas in contrast the addition of sodium nitrite at 250µM incrementally killed C albicans as the pH was reduced to below 4. The nitrite was in fact effective in eliminating C albicans at pH 1 at all concentrations above 250µM (data not shown). 5nN nitrite killed C albicans at up to pH5. It is significant that a random sample of 10 laboratory personnel on a normal diet had fasting salivary nitrite which varied from 23 to 220µM (mean 114µM) rising to 409 to 1890µM (mean 1030) 45 minutes after ingestion of 200mg potassium nitrate solution.

### EXAMPLE 2

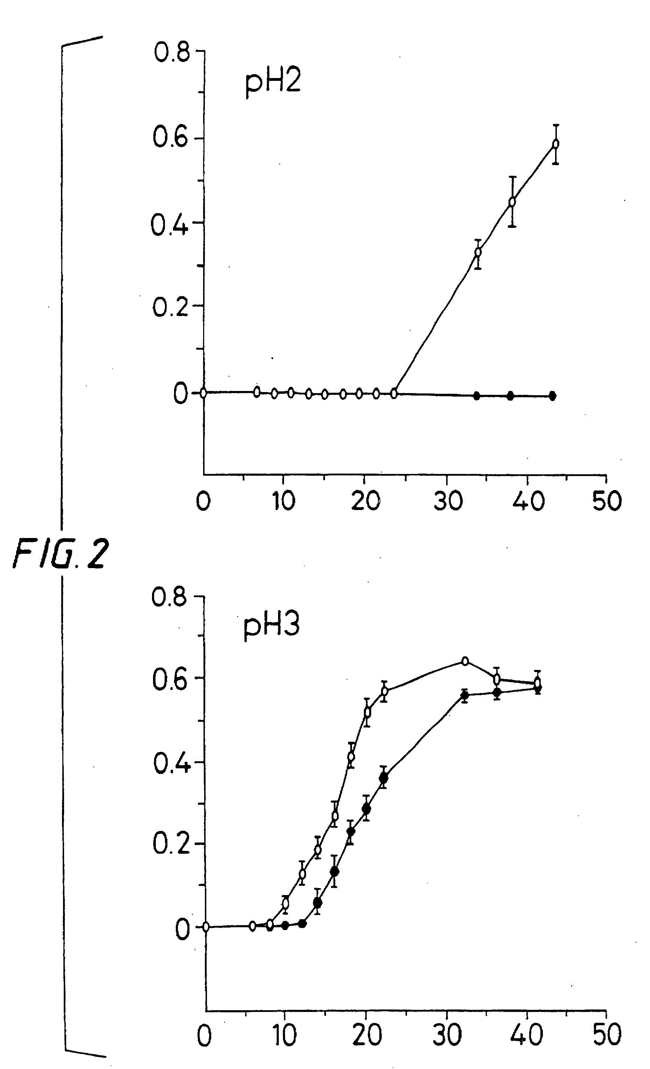

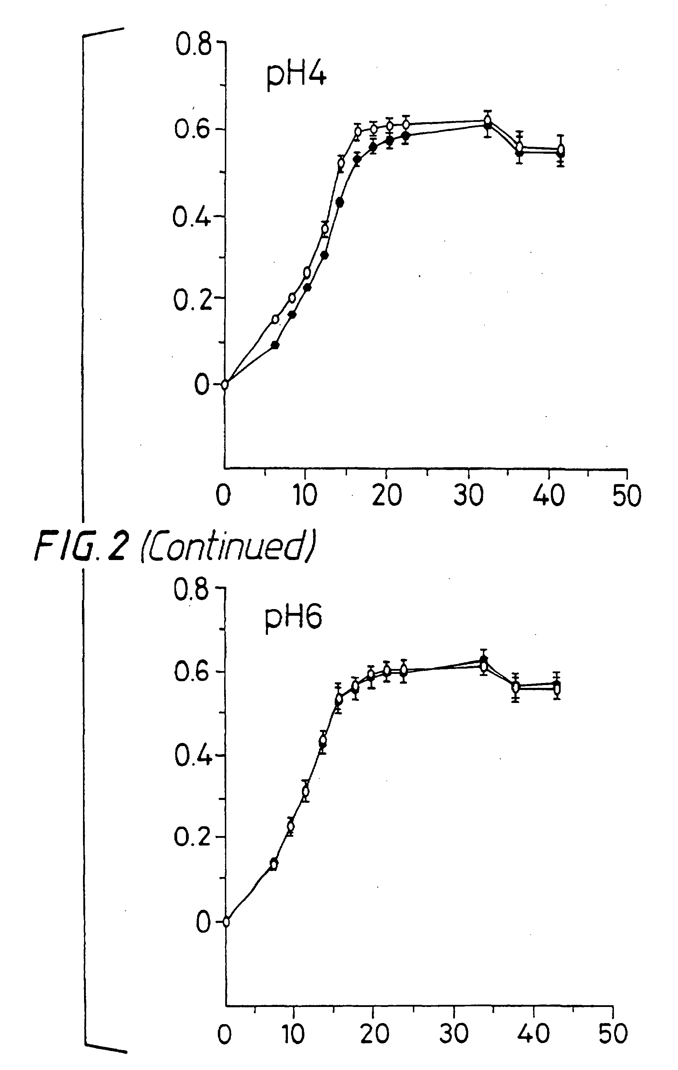

Figure 2 shows growth curves of E coli following exposure to acid alone (open symbols) or acid and 250µM nitrite (closed symbols). Growth was significantly (p≺0.05) impaired at pH 2,3 and 4 in the presence of nitrite compared with control.

The same methods were used as in Figure 1 except E coli (strain NCTC 10418 grown on MacConkey's agar) was used and nutrient both (Oxoid CM1) was used in place of Sabouraud's broth. The results shown in Figure 2 are a mean of 20 experiments. As can be seen from Figure 2 E coli is more susceptible to acid than C albicans. Nevertheless exposure to pH 2 for one hour does not kill all the organisms as there is significant growth in the nutrient broth. At pH3 many organisms survive. The addition of 250µM nitrite to the exposure medium eliminates E coli at pH2 and significantly reduces the viability of this organism at pH3 and pH4. Nitrite at this concentration had no effect above pH4.

### EXAMPLE 3

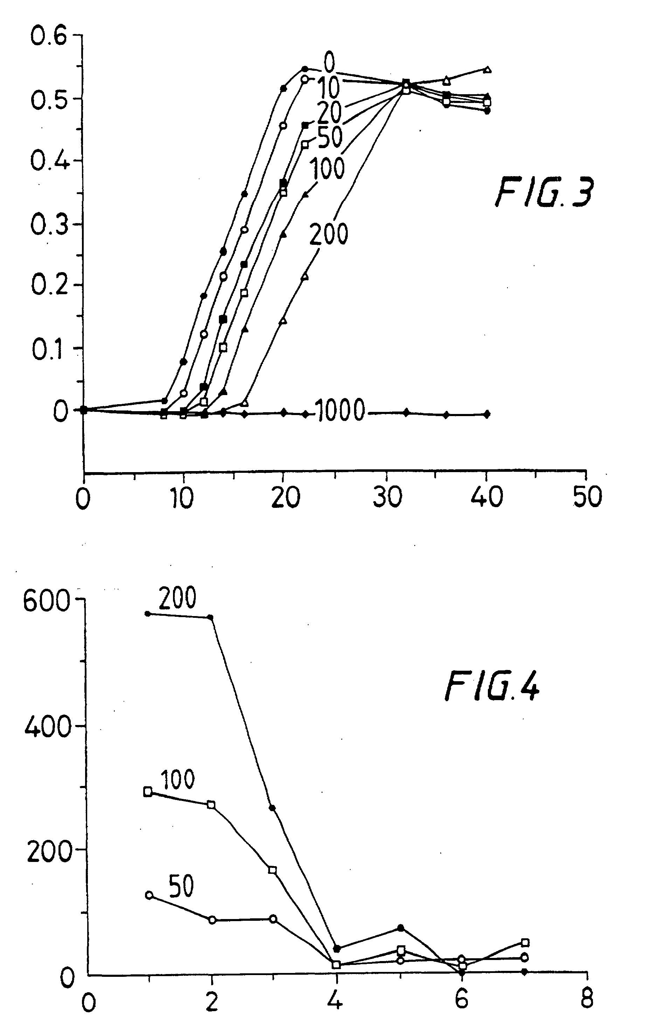

Figure 3 shows growth curves of E coli following exposure to pH3 in various nitrite concentrations (10-1000µM final concentration). The methods are those as for Figure 2. Figure 3 shows that there is a direct relationship between the toxic effects of nitrite on E coli and nitrate concentration at pH3. Even 10µM had a discernable effect whereas 1mM killed E coli completely.

### EXAMPLE 4

Figure 4 shows the generation of nitric oxide from sodium nitrite (as µM) at different acidities. Conditions were the same as those used for the exposure of organisms in Figure 1. In particular nitrite was added to citrate/phosphate buffer to achieve final concentrations shown in Figure 4. Nitric oxide concentrations in the buffer were measured by a nitric oxide sensitive meter (ISO-NO, World Precision Instruments) connected to a Maclab acquisition system and Macintosh computer. Measurements were recorded continually and readings were taken at 2 minutes when nitric oxide concentration had reached a steady state. Figure 4 shows the release of nitric oxide as a result of reducing pH. Nitric oxide, which we have shown is generated under experimental conditions in Figure 4 readily diffuses through cell membranes and has a high affinity for iron-sulphur containing respiratory enzymes and damages bacterial DNA. When produced enzymatically by activated leucocytes, nitric acid will kill Leishmania sp., Staphylococcus sp., Francisella sp. and Microbacterium as well as C albicans. Reaction with superoxide under acid conditions may additionally produce highly reactive hydroxyl radicals.

### EXAMPLE 5

In a study to investigate the effect of a combination of salicylic acid at 2% w/w and sodium nitrite at 2% w/w in 9 patient volunteers with microbiologically proven fungal infection of the feet, application of the treatment produced a microbiological cure in all but one patient after 2 weeks of therapy. The symptom score (derived from a scoring system which measures erythema, vesicles, pustules, desquamation, encrustation and pruritus) decreased from a mean of 7 before treatment to a mean of 2 following treatment.

### EXAMPLE 6

Investigation of the use of nitrate or nitrite administered topically in the mouth in the form of toothpaste, mouthwash or other orally acceptable vehicle to reduce the number of caries-producing organisms in dental plaque and to treat to prevent infection with C albicans or other harmful organisms showed such application to be effective.

The observation that oxides of nitrogen produced non-enzymatically from nitrite under conditions simulating those in the stomach kills C albicans and E coli extends these observations to the intestinal tract. E coli is closely related to Salmonella, Shigella and other pathogenic enterobacteria; all important causes of gastroenteritis in the mammal.

These results provide a rationale for active secretion of nitrate by the salivary glands. Nitrate itself is a innocuous precursor which only produces microbiocidal species when converted to nitrite and subjected to acid conditions. It is possible that Lactobacilli sp. transiently produce sufficient acid in the mouth after a carbohydrate meal to control the growth of oral pathogens but clearly a moderate intake of nitrate may be a desirable prerequisite in any contaminated environment despite any potential as a precursor of nitrosamines.

Further the production of intestinal nitrogen oxides may be inadequate if the oral flora which convert nitrate to nitrite are suppressed following therapy with broad-spectrum antibiotics. Similarly if gastric acid production is reduced, or if nitrate intake, which is largely dependent on leafy vegetables, is low this protective mechanism will be impaired. These are precisely the situations which predispose to oral and intestinal infections.

Whereas the foregoing study has concentrated on C albicans and E coli and the other organisms mentioned, it may also be important for providing protection from other serious gut pathogens which when swallowed may cause duodenal ulceration, for example Helicobacter pylori, amoebic dysentery and chronic intestinal parasitism. Accordingly the invention provides a dosage form for the treatment of bacterial, viral or fungal conditions, a method of sterilising an object, and a composition therefor.

The above also suggests an inexpensive and simple means of prevention of gastroenteritis in farmed pigs by modification of dietary nitrate intake without the use of antibiotics.

## Claims

1. An antimicrobial dosage form which comprises;
a pharmaceutically acceptable acidifying agent and a pharmaceutically acceptable source of nitrite or a nitrate precursor for said source of nitrite,
both said acidifying agent and said source of nitrite or nitrate precursor therefor being separately disposed in a respective pharmaceutically acceptable carrier or diluent for admixture to release nitrite at the intended environment of use,
said carrier or diluent being a cream or ointment, and
wherein the acidifying agent is present in an amount sufficient to reduce the pH at the environment of use to below pH4.

2. A dosage form according to claim 1, wherein the acidifying agent is an organic acid.

3. An antimicrobial dosage form which comprises;
a pharmaceutically acceptable source of nitrite or a nitrate precursor for said source of nitrite,
and a pharmaceutically acceptable carrier or diluent therefor,
the dosage form being acidified by means of an acidifying agent which is a pharmaceutically acceptable organic acid,
and wherein the nitrite, when mixed with the acid, produces antimicrobially effective oxides of nitrogen, the acid being present in an amount sufficient to reduce the pH at the environment of use to below pH4, and the nitrite is present in concentrations of up to 4%.

4. A dosage form according to claim 3, wherein the pharmaceutically acceptable carrier is disposed in an inert cream or ointment, and wherein said acid and said source of nitrite are separately disposed in a respective cream or ointment for admixture to release nitrite at the intended environment of use.

5. A dosage form according to any preceding claim, wherein the acidifying agent is salicylic acid.

6. A dosage form according to any of claims 1 to 4, wherein the acidifying agent is ascorbic acid.

7. A dosage form according to any preceding claim, wherein the nitrate precursor is an alkali metal or alkali earth metal nitrate.

8. A dosage form according to any of claims 3 or 5 to 7, in tablet or liquid form.

9. A method of sterilising an object which method comprises the steps of:-
1) preparing a pharmaceutically acceptable acidifying agent and a pharmaceutically acceptable source of nitrite or a nitrate precursor therefor,
2) admixing said acidifying agent with said source of nitrite in a liquid carrier or diluent in contact with said object, thereby to reduce the pH to below 4 to release sterilant nitrite to sterilise said object.

10. A method according to claim 9, wherein said acidifying agent is an organic acid.

11. A method according to claim 10, wherein said organic acid is a salicylic acid.

12. A method according to any of claims 9 to 11, wherein said precursor is an alkali metal or alkali earth metal nitrate.

13. A sterilant composition comprising a pharmaceutically acceptable acidifying agent,
a pharmaceutically acceptable form of nitrite or a precursor therefor, and
a pharmaceutically acceptable carrier or diluent therefor, wherein the acidifying agent is adapted to reduce the pH at the environment of use to below 4.

14. An animal feed supplement comprising a pharmaceutically acceptable acidifying agent, and a pharmaceutically acceptable source of nitrite or a nitrate precursor therefor, in an amount sufficient to produce a beneficial anti-bacterial effect but insufficient to produce an adverse reaction in a target animal.

15. An animal feed supplement according to claim 14, wherein the acidifying agent is selected from salicylic or ascorbic acid.

16. An animal feed supplement according to either of claims 14 or 15, wherein the source of nitrite is an inorganic nitrate.

17. An animal feed supplement according to claim 16, wherein the feed supplement is adapted for the pig, and the inorganic nitrate is present in the feed in an amount sufficient to provide an adult pig with about 1 g/day.

18. Use of a pharmaceutically acceptable organic acid, as defined in any of claims 3, 5 or 6, and a pharmaceutically acceptable source of nitrite or a nitrate precursor for said source of nitrite, as defined in claims 3 or 7, in the manufacture of an antimicrobial medicament, as defined in claims 3, 4 or 8, wherein the nitrite, when mixed with the acid, produces antimicrobially effective oxides of nitrogen, the acid being present in an amount sufficient to reduce the pH at the environment of use to below pH4, the nitrite being present in concentrations of up to 4%.

19. Use of a pharmaceutically acceptable acidifying agent, as defined in any of claims 1, 5 or 6, and a pharmaceutically acceptable source of nitrite or a nitrate precursor for said source of nitrite, as defined in claims 1 or 7, in the manufacture of an antimicrobial medicament, as defined in claims 1 or 8,
both said acidifying agent and said source of nitrite or nitrate precursor therefor being separately disposed in a respective pharmaceutically acceptable carrier or diluent for admixture to release nitrite at the intended environment of use,
said carrier or diluent being a cream or ointment, and
wherein the acidifying agent is present in an amount sufficient to reduce the pH at the environment of use to below pH4.

20. A dosage form according to any of claims 1 to 8, wherein the dosage form is for use in the treatment of a viral condition.

21. A dosage form according to any of claims 1 to 8, wherein the dosage form is for use in the treatment of a fungal condition.

22. A dosage form according to any of claims 1 to 8, wherein the dosage form is for use in the treatment of a bacterial condition.

## Patentansprüche

1. Antimikrobielle Dosierungsform, welche umfaßt;
ein pharmazeutisch verträgliches ansäuerndes Mittel und einen pharmazeutisch verträglichen Ausgangsstoff von Nitrit oder ein Nitratvorprodukt für den Ausgangsstoff von Nitrit,
wobei sowohl das ansäuernde Mittel als auch der Ausgangsstoff von Nitrit oder das Nitratvorprodukt dafür gesondert in einem entsprechenden pharmazeutisch verträglichen Träger oder Verdünnungsmittel verteilt sind, zum Mischen, um Nitrit in der vorgesehenen Umgebung der Verwendung freizusetzen,
wobei der Träger oder das Verdünnungsmittel eine Creme oder Salbe sind und
wobei das ansäuernde Mittel in einer Menge, ausreichend, um den pH in der Umgebung der Verwendung auf unter pH 4 zu verringern, vorhanden ist.

2. Dosierungsform nach Anspruch 1, wobei das ansäuernde Mittel eine organische Säure ist.

3. Antimikrobielle Dosierungsform, welche umfaßt;
einen pharmazeutisch verträglichen Ausgangsstoff von Nitrit oder ein Nitratvorprodukt für den Ausgangsstoff von Nitrit,
und einen pharmazeutisch verträglichen Träger oder ein Verdünnungsmittel dafür,
wobei die Dosierungsform mittels eines ansäuernden Mittels, welches eine pharmazeutisch verträgliche organische Säure ist, angesäuert wird,
und wobei das Nitrit, wenn mit der Säure gemischt, antimikrobiell wirksame Oxide von Stickstoff erzeugt, wobei die Säure in einer Menge, ausreichend, um den pH in der Umgebung der Verwendung auf unter pH 4 zu verringern, vorhanden ist und das Nitrit in Konzentrationen von bis zu 4% vorhanden ist.

4. Dosierungsform nach Anspruch 3, wobei der pharmazeutisch verträgliche Träger in einer inerten Creme oder Salbe verteilt ist und wobei die Säure und der Ausgangsstoff von Nitrit gesondert in einer entsprechenden Creme oder Salbe verteilt sind, zum Mischen, um Nitrit in der vorgesehenen Umgebung der Verwendung freizusetzen.

5. Dosierungsform nach einem vorhergehenden Anspruch, wobei das ansäuernde Mittel Salicylsäure ist.

6. Dosierungsform nach einem der Ansprüche 1 bis 4, wobei das ansäuernde Mittel Ascorbinsäure ist.

7. Dosierungsform nach einem vorhergehenden Anspruch, wobei das Nitratvorprodukt ein Alkalimetall- oder Erdalkalimetallnitrat ist.

8. Dosierungsform nach einem der Ansprüche 3 oder 5 bis 7 in Tablettenform oder in flüssiger Form.

9. Verfahren zum Sterilisieren eines Gegenstands, welches Verfahren die Schritte umfaßt:
1) Herstellen eines pharmazeutisch verträglichen ansäuernden Mittels und eines pharmazeutisch verträglichen Ausgangsstoffes von Nitrit oder eines Nitratvorprodukts dafür,
2) Mischen des ansäuernden Mittels mit dem Ausgangsstoff von Nitrit in einem flüssigen Träger oder Verdünnungsmittel in Kontakt mit dem Gegenstand, um dadurch den pH auf unter 4 zu verringern, um das Sterilisierungsmittel Nitrit freizusetzen, um den Gegenstand zu sterilisieren.

10. Verfahren nach Anspruch 9, wobei das ansäuernde Mittel eine organische Säure ist.

11. Verfahren nach Anspruch 10, wobei die organische Säure eine Salicylsäure ist.

12. Verfahren nach einem der Ansprüche 9 bis 11, wobei das Vorprodukt ein Alkalimetall- oder Erdalkalimetallnitrat ist.

13. Sterilisierungsmittelzusammensetzung, umfassend ein pharmazeutisch verträgliches ansäuerndes Mittel,
eine pharmazeutisch verträgliche Form von Nitrit oder ein Vorprodukt dafür und
einen pharmazeutisch verträglichen Träger oder ein Verdünnungsmittel dafür,
wobei das ansäuernde Mitte angepaßt ist, den pH in der Umgebung der Verwendung auf unter 4 zu verringern.

14. Tierfutterzusatz, umfassend ein pharmazeutisch verträgliches ansäuerndes Mittel und einen pharmazeutisch verträglichen Ausgangsstoff von Nitrit oder ein Nitratvorprodukt dafür in einer Menge, ausreichend, um eine vorteilhafte antibakterielle Wirkung zu erzeugen, aber unzureichend, um eine abträgliche Reaktion bei einem Testtier zu erzeugen.

15. Tierfutterzusatz nach Anspruch 14, wobei das ansäuemde Mittel aus Salicyl- oder Ascorbinsäure ausgewählt ist.

16. Tierfutterzusatz nach einem der Ansprüche 14 oder 15, wobei der Ausgangsstoff von Nitrit ein anorganisches Nitrat ist.

17. Tierfutterzusatz nach Anspruch 16, wobei der Futterzusatz für das Schwein angepaßt ist und das anorganische Nitrat in dem Futter in einer Menge, ausreichend, um ein erwachsenes Schein mit etwa 1 g/Tag zu versorgen, vorhanden ist.

18. Verwendung einer pharmazeutisch verträglichen organischen Säure, wie definiert in einem der Ansprüche 3, 5 oder 6, und eines pharmazeutisch verträglichen Ausgangsstoffes von Nitrit oder eines Nitratvorprodukts für den Ausgangsstoff von Nitrit, wie definiert in den Ansprüchen 3 oder 7, bei der Herstellung eines antimikrobiellen Medikaments, wie definiert in den Ansprüchen 3, 4 oder 8, wobei das Nitrit, wenn gemischt mit der Säure, antimikrobiell wirksame Oxide von Stickstoff erzeugt, wobei die Säure in einer Menge, ausreichend, um den pH in der Umgebung der Verwendung auf unter pH 4 zu verringern, vorhanden ist, wobei das Nitrit in Konzentrationen von bis zu 4% vorhanden ist.

19. Verwendung eines pharmazeutisch verträglichen ansäuernden Mittels, wie definiert in einem der Ansprüche 1, 5 oder 6, und eines pharmazeutisch verträglichen Ausgangsstoffes von Nitrit oder eines Nitratvorprodukts für den Ausgangsstoff von Nitrit, wie definiert in den Ansprüchen 1 oder 7, bei der Herstellung eines antimikrobiellen Medikaments, wie definiert in den Ansprüchen 1 oder 8,
wobei sowohl das ansäuernde Mittel als auch der Ausgangsstoff von Nitrit oder das Nitratvorprodukt dafür gesondert in einem entsprechenden pharmazeutisch verträglichen Träger oder Verdünnungsmittel verteilt sind, zum Mischen, um Nitrit in der vorgesehenen Umgebung der Verwendung freizusetzen,
wobei der Träger oder das Verdünnungsmittel eine Creme oder Salbe sind und
wobei das ansäuernde Mittel in einer Menge, ausreichend um den pH in der Umgebung der Verwendung auf unter pH 4 zu verringern, vorhanden ist.

20. Dosierungsform nach einem der Ansprüche 1 bis 8, wobei die Dosierungsform zur Verwendung bei der Behandlung eines viralen Leidens bestimmt ist.

21. Dosierungsform nach einem der Ansprüche 1 bis 8, wobei die Dosierungsform zur Verwendung bei der Behandlung eines fungalen Leidens bestimmt ist.

22. Dosierungsform nach einem der Ansprüche 1 bis 8, wobei die Dosierungsform zur Verwendung bei der Behandlung eines bakteriellen Leidens bestimmt ist.

## Revendications

1. Une forme posologique à activité antimicrobienne qui comprend:
un agent acidifiant pharmaceutiquement acceptable et une source pharmaceutiquement acceptable de nitrite ou de nitrate précurseur de ladite source de nitrite,
ledit agent acidifiant et ladite source de nitrite ou de nitrate précurseur de nitrite étant séparément répartis dans des véhicules ou diluants pharmaceutiquement acceptables distincts pour mélange afin de libérer le nitrite dans l'environnement d'utilisation prévu,
ledit véhicule ou diluant correspondant à une crème ou une pommade, et
dans laquelle l'agent acidifiant est présent en une quantité suffisante pour abaisser le pH de l'environnement d'utilisation à moins de pH 4.

2. Une forme posologique selon la revendication 1, dans laquelle l'agent acidifiant est un acide organique.

3. Une forme posologique à activité antimicrobienne qui comprend:
une source pharmaceutiquement acceptable de nitrite ou de nitrate précurseur de ladite source de nitrite,
et un véhicule ou diluant pharmaceutiquement acceptable de cette source,
la forme posologique étant acidifiée au moyen d'un agent acidifiant qui est un acide organique pharmaceutiquement acceptable,
et dans laquelle le nitrite, quand il est mélangé à l'acide, produit des oxydes d'azote à activité antimicrobienne efficace, l'acide étant présent en une quantité suffisante pour abaisser le pH de l'environnement d'utilisation à moins de pH 4 et le nitrite étant présent à des concentrations allant jusqu'à 4% au maximum.

4. Une forme posologique selon la revendication 3, dans laquelle le véhicule pharmaceutiquement acceptable est réparti dans une crème ou une pommade inerte, et dans laquelle ledit acide et ladite source de nitrite sont séparément répartis dans des crèmes ou pommades distinctes qui sont mélangées pour libérer le nitrite dans l'environnement d'utilisation prévu.

5. Une forme posologique selon l'une quelconque des revendications précédentes, dans laquelle l'agent acidifiant est l'acide salicylique.

6. Une forme posologique selon l'une quelconque des revendications 1 à 4, dans laquelle l'agent acidifiant est l'acide ascorbique.

7. Une forme posologique selon l'une quelconque des revendications précédentes, dans laquelle le nitrate précurseur est le nitrate d'un métal alcalin ou d'un métal alcalino-terreux.

8. Une forme posologique selon l'une quelconque des revendications 3 ou 5 à 7, en comprimés ou sous une forme liquide.

9. Un procédé de stérilisation d'un objet, qui comporte les étapes consistant à:
1) préparer un agent acidifiant pharmaceutiquement acceptable et une source de nitrite ou de nitrate précurseur de celle-ci pharmaceutiquement acceptable,
2) mélanger ledit agent acidifiant avec ladite source de nitrite dans un véhicule ou diluant liquide en contact avec ledit objet pour produire un abaissement du pH à moins de 4 et libérer le nitrite stérilisant pour stériliser ledit objet.

10. Un procédé selon la revendication 9, dans lequel ledit agent acidifiant est un acide organique.

11. Un procédé selon la revendication 10, dans lequel ledit acide organique est l'acide salicylique.

12. Un procédé selon l'une quelconque des revendications 9 à 11, dans lequel ledit précurseur est le nitrate d'un métal alcalin ou d'un métal alcalino-terreux.

13. Une composition stérilisante comprenant un agent acidifiant pharmaceutiquement acceptable,
un nitrite ou précurseur de nitrite sous une forme pharmaceutiquement acceptable, et
un véhicule ou diluant de celui-ci pharmaceutiquement acceptable, l'agent acidifiant étant adapté de façon à abaisser le pH de l'environnement d'utilisation à moins de 4.

14. Un supplément diététique pour animaux comprenant un agent acidifiant pharmaceutiquement acceptable et une source pharmaceutiquement acceptable de nitrite ou de nitrate précurseur de ladite source de nitrite en une quantité suffisante pour produire un effet antibactérien favorable mais trop basse pour induire des réactions indésirables chez l'animal visé.

15. Un supplément diététique pour animaux selon la revendication 14, dans lequel l'agent acidifiant est sélectionné parmi l'acide salicylique et l'acide ascorbique.

16. Un supplément diététique pour animaux selon la revendication 14 ou 15, dans lequel la source de nitrite est un nitrate inorganique.

17. Un supplément diététique pour animaux selon la revendication 16 qui est adapté aux porcins et qui contient le nitrate inorganique en une quantité suffisante pour que les apports fournis à un porc adulte soit de l'ordre de 1 g/jour.

18. L'utilisation d'un acide organique pharmaceutiquement acceptable tel que défini à l'une quelconque des revendications 3, 5 ou 6 et d'une source pharmaceutiquement acceptable de nitrite ou de nitrate précurseur de ladite source de nitrite telle que définie à l'une quelconque des revendications 3 ou 7 dans la fabrication d'un médicament à activité antimicrobienne tel que défini dans l'une quelconque des revendications 3, 4 ou 8 dans lequel le nitrite, quand il est mélangé à l'acide, produit des oxydes d'azote à activité antimicrobienne efficace, l'acide étant présent en une quantité suffisante pour abaisser le pH de l'environnement d'utilisation à moins de pH 4 et le nitrite étant présent à des concentrations allant jusqu'à 4 % au maximum.

19. L'utilisation d'un agent acidifiant pharmaceutiquement acceptable tel que défini à l'une quelconque des revendications 1, 5 ou 6 et d'une source pharmaceutiquement acceptable de nitrite ou de nitrate précurseur de ladite source de nitrite telle que définie à l'une quelconque des revendications 1 ou 7 dans la fabrication d'un médicament à activité antimicrobienne tel que défini dans l'une quelconque des revendications 1 ou 8,
ledit agent acidifiant et ladite source de nitrite ou de nitrate précurseur de nitrite étant séparément répartis dans des véhicules ou diluants pharmaceutiquement acceptables distincts pour mélange afin de libérer le nitrite dans l'environnement d'utilisation prévu,
ledit véhicule ou diluant correspondant à une crème ou une pommade, et
dans laquelle l'agent acidifiant est présent en une quantité suffisante pour abaisser le pH de l'environnement d'utilisation à moins de pH 4.

20. Une forme posologique selon l'une quelconque des revendications 1 à 8, qui est indiquée dans le traitement d'une affection virale.

21. Une forme posologique selon l'une quelconque des revendications 1 à 8, qui est indiquée dans le traitement d'une affection fongique.

22. Une forme posologique selon l'une quelconque des revendications 1 à 8, qui est indiquée dans le traitement d'une affection bactérienne.
